# EUROPEAN PATENT SPECIFICATION

(11) **EP 1 563 831 B1**
(45) Date of publication and mention of the grant of the patent: **02.09.2015**
(21) Application number: 04801807.1
(22) Date of filing: 26.10.2004
(51) Int. Cl.: A61Q 19/08, A61Q 19/00, A61K 8/81, A61K 8/88

(54) **EXTERNAL COMPOSITION FOR SKIN**
ÄUSSERLICHE ZUSAMMENSETZUNG FÜR HAUT
PREPARATION POUR LA PEAU A USAGE EXTERNE

(43) Date of publication of application: 17.08.2005
(73) Proprietor: Shiseido Co., Ltd., Tokyo 104-8010 (JP)
(72) Inventor: OMURA, Takayuki, SHISEIDO RESEARCH CENTER, Yokohama-shi, Kanagawa 2248558 (JP); MATSUO, Ayano, SHISEIDO RESEARCH CENTER, Yokohama-shi, Kanagawa 2248558 (JP); HIWATARI, Yoshiko, SHISEIDO RESEARCH CENTER, Yokohama-shi, Kanagawa 2248558 (JP); KANEDA, Isamu, SHISEIDO RESEARCH CENTER, Yokohama-shi, Kanagawa 2248558 (JP)
(74) Representative: Gudat, Axel
(86) International application number: PCT/JP2004/015831
(87) International publication number: WO 2005/044216

(56) References cited:
- WO-A-01/12146
- JP-A- 2000 119 233
- JP-A- 2001 072 764
- JP-A- 2001 354 542
- JP-A- 2003 012 442
- JP-A- 2004 143 064
- JP-A- 2004 149 467
- JP-A- 2004 532 290
- DATABASE WPI Week 200436 Derwent Publications Ltd., London, GB; AN 2004-380595 XP002483618 & JP 2004 107227 A (KAO CORP) 8 April 2004 (2004-04-08)
- DATABASE WPI Week 200382 Derwent Publications Ltd., London, GB; AN 2003-881936 XP002483620 & JP 2003 146830 A (SAKATA K) 21 May 2003 (2003-05-21)
- DATABASE WPI Week 200141 Derwent Publications Ltd., London, GB; AN 2001-384466 XP002483621 & JP 2001 072764 A (MITSUI CHEM INC) 21 March 2001 (2001-03-21)

## Description

### FIELD OF THE INVENTION

The present invention relates to an external composition for skin, and in particular, to an external composition for skin containing an acrylic polymer.

### BACKGOUND OF THE INVENTION

Conventionally, moisturizing agents and film-forming agents are blended to an external composition for skin for the purpose of improving elasticity and alleviating wrinkles of skin. The moisturizing agent supplies water to surface and interior of skin and it provides elasticity to skin. However, unless a relatively large amount is blended, it is not effective and it may cause heavy sticky feeling.

On the other hand, the film-forming agent stretches fine wrinkles with the force of contraction, which is generated during film formation, provides elasticity to skin, and temporarily removes wrinkles. However, there are problems in that the adhesion to skin is weak and use may be uncomfortable.

In order to improve elasticity and alleviating wrinkles and to achieve good moisturizing, cosmetics containing combination of thickeners such as carboxyvinyl polymers, xanthan gum, hydroxyethyl cellulose, and moisturizing agents such as glycerin have been disclosed (Japanese Patent Application Laid-Open (Kokai) NO. 2000-143477, and Japanese Patent Application Laid-Open (Kokai) NO. 2004-149463).

However, the above-described cosmetics had problems in that the spreadability on skin was poor, or they became sticky after application. In addition, good moisturizing effect could not be achieved, and the effect of improving elasticity and alleviating wrinkles was not sufficient.

JP 2004-107227-A and WO 01/12146-A disclose cosmetic formulations having a good spreadability and comprising Aculyn®22 (acrylates/steareth-20 methacrylate copolymer) and a moisturizing agent. JP 2003-146830-A discloses a cosmetic formulation having a good moisturizing effect and comprising cross-linked poly-gamma-glutamic acid. Methods for preparing cross-linked poly amino acids are disclosed in JP 2001 072764-A, JP 2001 354542-A, JP 2003 012442-A, JP 2004 149467-A, JP 2000 119233-A, and 2004 532290-A.

### SUMMARY OF THE INVENTION

The present invention has been made in view of the above-described problems of the prior art. An object of the present invention is to provide an external composition for skin that has excellent effects in improving elasticity and alleviating wrinkles while moisturizing the skin, has good spreadability on the skin, and has no stickiness.

In view of the above circumstances, the present inventors have diligently carried out an investigation. As a result, the inventors have found that the following external composition for skin can be prepared. This external composition for skin has excellent effects in improving elasticity and alleviating wrinkles while moisturizing the skin, has good spreadability on the skin, and has no stickiness. The present invention was achieved by using a specific acrylic polymer as a thickener and by blending a cross-linked polyamino acid.

The external composition for skin of the present invention is characterized by comprising:
(A) one or more selected from acrylate/ceteth-20 methacrylate copolymer, acrylate/steareth-50 methacrylate copolymer, acrylate/steareth-20 methacrylate copolymer, and acrylate/beheneth-25 methacrylate copolymer and,
(B) one or more selected from cross-linked poly-gamma-glutamic acid, cross-linked poly-aspartic acid and their salts.

In the above-mentioned external composition for skin of the present invention, it is preferable that the blending quantity of component (A) is from 0.01 to 10.0 weight % as a polymer, and that the blending quantity of component (B) is from 0.0001 to 5.0 weight % as a polymer.

In the above-mentioned external composition for skin, it is preferable that a water-soluble drug is comprised.

In the above-mentioned external composition for skin, it is preferable that pH is 6.0 to 7.5.

### BRIEF DESCRIPTION OF THE DRAWINGS

Fig. 1 shows the result of elasticity-improving test in this invention.
Fig. 2 shows the result of wrinkle-alleviating test in this invention.
Fig. 3 shows the result of moisturizing test in this invention.

### DESCRIPTION OF THE PREFERRED EMBODIMENT

The working mode of this invention is explained below.

### <Component (A)>

The acrylic polymer used in this invention is acrylates/ceteth-20 methacrylate copolymer, acrylates/steareth-50 methacrylate copolymer, acrylates/steareth-20 methacrylate copolymer, acrylates/beheneth-25 methacrylate copolymer, which are listed on the INCI (International Cosmetic Ingredient Dictionary). These copolymers are commercially available in water dispersion (polymer emulsion) under the product names Aculyn 22, Aculyn 28, etc. (Rohm & Haas).

Among all the acrylic polymer of this invention, especially ACULYN 22 (acrylate/steareth-20 methacrylate copolymer) is desirable, because it is not sticky to the touch. ACULYN 22 is water dispersion of copolymer of
(a1) methacrylic acid,
(a2) ethyl acrylate, and
(a3) ester between methacrylic acid and polyoxyethylene (20) stearyl ether.

Water dispersions of the above acrylic polymers are diluted with water etc. as necessary, and neutralized by the addition of alkali in order to thicken the dispersions. Therefore it is suitable that pH of external composition for skin in this invention is within the range of 6 to 7.5. As an alkali agent for neutralization, not restricted especially, inorganic bases such as sodium hydroxide, potassium hydroxide and so on, organic base such as triethanolamine, isopropanol amine, basic amino acid and so on can be used.

In the external composition for skin of the present invention, the blending quantity of the above component (A) is preferably from 0.01 to 10.0weight % as a polymer, more preferably from 0.03 to 9.0 weight %, further more preferably from 0.05 to 5.0 weight %. If the quantity is less than 0.01 weight %, the effect of improving elasticity and alleviating wrinkles cannot be sufficiently achieved. If the quantity exceeds 10.0 weight %, the utilization is impaired.

The thickening action of the above component (A) is mainly due to the hydrophobic action of the hanging alkyl group on the tip of the POE chain and the positive charge repulsion by the carboxyl group.

### <Component (B)>

The cross-linked polyamino acid has a structure in which part of the polyamino acid is cross-linked. The basic skeleton of the cross-linked polyamino acid consists of polypeptides formed by condensation of amino acids.

The cross-linked polyamino acid used in the present invention is cross-linked polyaspartic acid or cross-linked poly-γ-glutamic acid. A homopolymer of poly-γ-glutamic acid is especially desirable.

The cross-linked polyamino acid of the present invention can be of a salt form. As counter ion of carboxyl group, alkali metal (Na, K etc.) salt, ammonium salt, amine salt and so on are listed. As a salt, sodium salt or potassium salt is desirable from a viewpoint of smell.

### (Manufacturing process of cross-linked polyamino acid)

There is no special restriction for the manufacturing process of cross-linked polyamino acids. For example, they can be prepared by publicly known methods described in Japanese Patent Application Laid-Open (Kokai) NO. Hei 7-224163, Japanese Patent Application Laid-Open (Kokai) NO. Hei 7-309943, Japanese Patent Application Laid-Open (Kokai) NO. 2001-72764, Japanese Patent Application Laid-Open (Kokai) NO. 2003-12442, etc. Especially in the present invention, cross-linked poly-γ-glutamic acid obtained by radiation cross-linking, which is described in Japanese Patent Application Laid-Open (Kokai) NO. 2003-12442, and the salt thereof is desirable from a viewpoint of feeling during use.

The concrete manufacturing process of cross-linked poly-gamma-glutamic acid by radiation-induced crosslinking is as follows.
1. Initially, the starting material poly-γ-glutamic acid is prepared. Poly-γ-glutamic acid can be prepared by various methods, for example, cultivation of microorganisms, namely cultivation of *Bacillus subtilis*, cultivation of genetically modified microorganisms, preparation with natto, or chemical synthesis.

For the preparation of poly-γ-glutamic acid by the microorganism cultivation, any strain is usable so far as it can produce poly-γ-glutamic acid outside of the bacteria. In particular, bacterial species of the genus *Bacillus* are desirable. Specific examples are *Bacillus subtilis, Bacillus anthracis, Bacillus natto*, and the like. Especially, poly-γ-glutamic acid, which is produced by microorganisms such as *Bacillus subtilis*, with a molecular weight of more than several millions is desirable.

So far as they can produce poly-γ-glutamic acid, any strain, culture medium, etc. can be used in the microorganism cultivation. For example, either a synthetic medium or natural medium can be used as a culture medium so far as the medium contains an appropriate carbon source, nitrogen source, inorganics, and other nutrients. Amino acids to be added include L-glutamic acid, aspartic acid, alanine, leucine, phenylalanine, histidine, etc., or salts thereof can be used. Preferably, L-glutamic acid is used from 2 to 12 weight %, and more preferably from 3 to 10 weight %.

As carbon source, glucose, sucrose, citric acid, xylose and so on can be used, among them citric acid or glucose is desirable. As nitrogen source, organic nutrition resource such as peptone or yeast extract, inorganic nutrition resource such as ammonium sulfate and so on can be used. The cultivation is carried out by shaking cultivation, stirring cultivation, and the like under aerobic conditions. The culture temperature is from 25 to 45 °C, preferably from 30 to 40 °C. The pH during the cultivation is from 5 to 9, preferably from 6 to 8. The pH during the cultivation is adjusted with sodium hydroxide, potassium hydroxide, and the like.

The cultivation is usually carried out for 48 to 72 hours, and the produced poly-γ-glutamic acid is accumulated outside of the bacteria. After the cultivation is completed, poly-γ-glutamic acid in the culture broth can be collected by the conventional method. That is, poly-γ-glutamic acid can be collected by centrifuging, filtering with a filter aid or a fine pore filter to remove bacteria, and by ultrafiltration. Then poly-γ-glutamic acid is precipitated by the addition of 3 to 4 times of ethanol. The precipitate is dissolved in water, insolubles are removed, and low molecular compounds are removed by dialysis or ultrafiltration etc. Poly-γ-glutamic acid can be collected by repeated reprecipitation by ethanol etc.

2. The above-described poly-γ-glutamic acid is dissolved in a solvent in the concentration range of 2 to 20weight %, preferably 5 to 15weight %. The obtained solution is irradiated, and the formed cross-linked compound is separated and purified. As solvent, acetone, methyl acetate, ethyl acetate and so on are used in addition to water and alcohol, and among them water, methyl alcohol and ethyl alcohol are desirable, especially water is desirable.

A radiation-permeable vessel such as a glass vial is used for a solution containing dissolved poly-γ-glutamic acid. There is no restriction for the type of radiation, for example, α-ray, β-ray, γ-ray, electron beam, neutron beam, or X-ray can be used. Preferably, the electron beam is used. The normal quantity of irradiated electron beam is preferably not less than 20 kGy. The irradiation time is preferably not less than 1 second. If it is less than 1 second, the formation of the cross-linked compound may not be sufficient.

Subsequently, solid cross-linked poly-γ-glutamic acid is obtained by removing the solvent. The poly-γ-glutamic acid is colorless and transparent, and it has excellent water-absorbing property and biodegradability. Poly-γ-glutamic acid obtained by the above method may be granulated to a predetermined shape or shaped into an undefined crushed form, spherical form, etc.

In the present invention, the blended amount of the above-described component (B) is, in terms of dry weight, 0.001 to 5.0 weight %, preferably 0.01 to 3.0 weight %, more preferably 0.2 to 3.0 weight %. If the amount is less than 0.001 weight %, the moisturizing effect is not sufficient. If the content exceeds 5.0 weight %, not only the moisturizing effect is not enhanced but also the following problems are created. These problems include time-dependent instability of the external composition for skin, stickiness, and heaviness, which are encountered during use.

In the present invention, external composition for skin comprising the above-described component (A) and component (B) are prepared. Thus, cosmetics that have no stickiness during use, good spreadability and adaptability to the skin, no stickiness after application, nice touch, excellent moist feeling, and excellent effects in improving elasticity and alleviating wrinkles can be obtained.

### <Water-soluble drug>

In the present invention, if water-soluble drugs, with anti-aging effects, such as thiotaurine, rose apple leaf extract, yeast extract, or rose extract are blended with the above-described component (A) and component (B), these drugs can be effectively infiltrated into the skin.

Also, a whitening agent can be blended. For example, as ascorbic acid line whitening agent, inorganic salt esters of ascorbic acid such as L-ascorbic acid, L-ascorbyl monophosphate, L-ascorbyl-2-sulfate, dl-alpha-tocopherol 2-L-ascorbyl diphosphate ; monoalkyl esters of ascorbic acid such as L-ascorbyl monostearate, L-ascorbyl monopalmitate, L-ascorbyl monooleate ; diesters of ascorbic acid such as L-ascorbyl distearate, L-ascorbyl dipalmitate, L-ascorbyl dioleate ; triesters of ascorbic acid such as L-ascorbyl tristearate, L-ascorbyl tripalmitate, L-ascorbyl trioleate ; ascorbic acid-2-glycosides such as 2-O-α-D-glucopyranosyl-L-ascorbic acid and so on can be listed.

The above-listed ascorbic acid and its derivatives are often blended as salts. Alkali metal salts (Na salt, K salt, etc.), alkaline earth metal salts (Ca salt, Mg salt, etc.), ammonium salts, alkanolamine salts, amino acid salts, etc. can be used, however, alkali metal salts are preferable.

As an example of alkoxysalicylic acid line whitening agent, for example, the one described in Japanese Patent Application Laid-Open (Kokai) NO. Hei 6-40886 is raised. As embodiments of said whitening agent, 3-methoxy salicylic acid, 3-ethoxy salicylic acid, 4-methoxy salicylic acid, 4-ethoxy salicylic acid, 4-propoxy salicylic acid, 4-isopripoxy salicylic acid, 4-buthoxy salicylic acid, 5-methoxy salicylic acid, 5-ethoxy salicylic acid, 5-propoxy salicylic acid, and salts of them can be listed. As salt, alkali metal salt (Na salt, K salt etc.), alkaline earth metal salt (Ca salt, Mg salt etc.), ammonium salt, alkanolamine salt, amino acid salt and so on are raised, among them alkali metal salt is desirable.

When a salt-type whitening agent is used in the external composition for skin of the present invention, it can be blended as a salt form or it can be neutralized with an alkaline agent in the composition. There is no restriction for alkaline agents used for the neutralization of salt-type whitening agents so far as they can form salts. For example, metal hydroxide such as sodium hydroxide, potassium hydroxide and so on; alkanolamine such as monoethanolamine, diethanolamine, triethanolamine; organic acid salt such as sodium citrate, potassium malate, sodium lactate and so on; amino acid such as lysine and so on can be listed. Among them, alkali metal hydroxide of sodium hydroxide and potassium hydroxide are desirable.

In this invention, the stability of the above salt-type whitening agent is guaranteed, by adjusting pH of external composition for skin at the range of 6 to 7.5.

Also, various kinds of medicines such as amino acid such as glycine, alanine, valine, leucine, threonine, phenylalanine, tyrosine, aspartic acid, asparagine, glutamine, taurine, arginine, histidine and so on and also these alkali metal salts and hydrochlorides ; acyl sarcosine acid (for example sodium lauroyl sarcosine), glutathione ; organic acid such as citric acid, malic acid, tartaric acid, lactic acid and so on ; Vitamin A and also the derivatives ; Vitamin B and the like such as vitamin B6 hydrochloride, vitamin B6 tripalmitate, vitamin B6 dioctanoate, vitamin B2 and also the derivatives, vitamin B12, vitamins B15 and also the derivative and so on ; Vitamin E and the like such as alpha-tocopherol, beta-tocopherol, gamma-tocopherol, vitamin E acetate and so on ; Vitamin D and the like; the vitamins such as vitamin H, pantothenic acid, pantethine and so on ; nicotinamide, benzyl nicotate, gamma-orizanol, allantoin, glycyrrhizinic acid (salt), glycyrrhetinic acid and also the derivatives, hinokitiol, 1-methyl-4-(1-hydroxy-1,5,5-trimethyl-4-pentenyl) cyclohex-1-ene, eucalyptol, thymol, inositol, saponins such as saikosaponin, carrot saponin, sponge cucumber saponin, soapberry saponin, pantothenyl ethyl ether, ethynyl estradiol, tranexamic acid, arbutins, cepharanthine, placenta extract can be listed.

There is no restriction for water-soluble drugs used so far as they can be usually blended in cosmetics. For example, angelica extract, gambir extract, althea extract, arnica extract, aloe extract, aloe vera extract, ginkgo extract, nettle extract, foeniculum extract, rose fruit extract, isodon japonicus extract, scutellaria root extract, phellodendron Bark extract, Coptis extract, Hypericum extract, Netherlands mustard extract, seaweed extract, Artemisiae Folium extract, brown alga extract, chamomile extract, wild oat extract, Artemisia capillaris extract, gardenia extract, striped bamboo extract, sophora root extract, clematis extract, cranesbill extract, tea extract, burdock extract, rice bran extract, comfrey extract, cactus extract, salvia extract, hawthorn extract, rehmannia root extract, perilla extract, filipendula extract, paeonia lactiflora extract, houttuynia herb extract, tincture zingiberis, iris root extract, white birch extract, water-soluble lithospermum root extract, ivy extract, yarrow extract, peppermint extract, peepul extract, mallow extract, swertia japonica extract, Mori Cortex extract, soybean extract, thymus extract, Thymus species extract, tea extract, clove extract, citrus unshiu peel extract, tincture capsici, Japanese angelica root extract, calendula extract, bitter orange peel extract, carrot extract, wild rose extract, parsley extract, witch hazel extract, rose extract, loquat extract, grape leaf extract, sponge cucumber extract, safflower extract, Typha angustata Bory et Chaub extract, Paeonia Suffruticosa extract, hop extract, marmelo extract, marronnier extract, rosemary extract, balm mint extract, sweet clover extract, peach leaf extract, cornflower extract, saxifrage extract, eucalyptus extract, lily extract, coix extract, lavender extract, lemon extract, rosemary extract, Rome chamomile extract, burnet extract, kiwi extract, grape fruit extract, royal jelly extract can be listed.

In the external composition for skin of this invention, even if surfactant is not blended, a sufficiently stable composition is formed. Nevertheless surfactant can be blended for the purpose of more improvement of stability of composition, or other purpose.

For example, nonionic surfactants such as sorbitan monolaurate, sorbitan monopalmitate, sorbitan sesqui oleate, sorbitan trioleate, polyoxyethylene sorbitan monolaurate, polyoxy ethylene glycol monooleate, polyoxyethylene alkyl ether, polyethylene glycol di-fatty acid ester, lauroyl diethanol amide, atty acid dipropanol amide, maltitol hydroxy aliphatic ether, alkylated polysaccharide, alkyl glucoside, sugar ester, polyether denatured silicone ; cationic surfactants such as stearyltrimethylammonium chloride, benzalkonium chloride, lauryl amine oxide ; anionic surfactants such as sodium palmitate, sodium laurate, sodium lauryl sulfate, potassium lauryl sulfate, triethanolamine alkylsulfate, acylmethyltaurine salt; ampholytic surfactants and so on can be blended by properly selecting.

In addition to the above-described components, other components that usually can be blended into the external use compositions such as cosmetics can be blended into the external composition for skin of the present invention so far as the desired effect of the present invention is not interfered.

For example, hydrocarbons such as liquid paraffin, squalane, vaseline; fats and oils such as macadamia nut oil, olive oil, lanolin; waxes such as jojoba oil, carnauba wax, candelilla wax; silicones such as dimethylpolysiloxane, methyl phenyl siloxane; higher alcohols such as capryl alcohol, lauryl alcohol, myristyl alcohol, cetyl alcohol, stearyl alcohol, behenyl alcohol, cholesterol, phytosterol; higher fatty acids such as capric acid, myristic acid, palmitic acid, stearic acid, behenic acid, lanolin fatty acid, linoleic acid, linolenic.; moisturizing agents such as polyethylene glycol, glycerol, 1,3-;the of butylene glycol, erythritol, sorbitol, xylitol, maltitol, mucopolysaccharides, hyaluronic acid, chondroitin sulfate, chitin, chitosan; lower alcohols such as ethanol; anti-oxidants such as butyl hydroxy toluene, tocopherol, phytin, antibacterial agents such as benzoic acid, salicylic acid, sorbic acid, paraoxy benzoic acid alkyl esters, hexachlorophene, coating agents, oil gelling agents, metal oxides, organic ultraviolet absorbents, inorganic metal salts, organic metal salts, alcohols, chelate agents, pH modifiers, antiseptics, thickeners, purified water and so on can be blended in the external composition of this invention properly.

There is no special restriction for the application form of the external composition for skin of the present invention, and any application form is allowed. For example, aqueous solution type, solubilization type, emulsification type, powder dispersion type, water-oil 2 layer type, water-oil-powder 3 layer type, oil/water (O/W) emulsification type, water/oil (W/O) emulsification type, gel, aerosol, mist and capsule can be listed. Products can also be of any type so far as the type is conventionally used for external composition for skin. They are, for example, facial cosmetics such as lotion, milky emulsion, cream, and pack; make-up cosmetics such as foundation, blusher, lipstick, eye shadow, eye liner, mascara, and sunscreen; body cosmetics; fragrance cosmetics; skin cleansing cosmetics such as make-up cleanser, face cleanser, and body shampoo; hair care cosmetics such as hair rinse and shampoo; ointment; and bath preparation.

The present invention is further described in the following Examples.

An amount is represented as weight % unless otherwise specified.

### [Activity of improving elasticity and alleviating wrinkles]

Each sample was applied to the faces of 10 professional panel members. Five hours after the application, the sensory evaluation was performed concerning the effectiveness in improving elasticity and alleviating wrinkles.

### (Valuation basis)

A: All 10 members answered that it was effective in improving elasticity and alleviating wrinkles.

B: 7 to 9 members answered that it was effective in improving elasticity and alleviating wrinkles.

C: 3 to 6 members answered that it was effective in improving elasticity and alleviating wrinkles.

D: 2 or less members answered that it was effective in improving elasticity alleviating and wrinkles.

### [Moisturizing activity]

Each sample was applied to the faces of 10 professional panel members. The sensory evaluation was performed concerning the moist feeling (moisturizing effect) after the application.

### (Valuation basis)

A: All 10 members answered that it had moist feeling.
B: 7 to 9 members answered that it had moist feeling.
C: 3 to 6 members answered that it had moist feeling.
D: 2 or less members answered that it had moist feeling.

### [Stickiness]

Each sample was applied to the faces of 10 professional panel members. The sensory evaluation was performed concerning the stickiness after the application.

### (Valuation basis)

A: All 10 members answered that it had no stickiness.
B:7 to 9 members answered that it had no stickiness.
C:3 to 6 members answered that it had no stickiness.
D: 2 or less members answered that it had no stickiness.

### [Spreadability on skin]

Each sample was applied to the faces of 10 professional panel members. The sensory evaluation was performed concerning the spreadability at the application.

### (Valuation basis)

A: All 10 members answered that it had easy spreadability and there was a smooth feeling upon use.
B: 7 to 9 members answered that it had easy spreadability and there was a smooth feeling upon use.
C: 3 to 6 members answered that it had easy spreadability and there was a smooth feeling upon use.
D: 2 or less members answered that it had easy spreadability and there was a smooth feeling upon use.

### (Manufacturing example 1) Cross-linked poly-gamma-glutamic acid

10weight % aqueous solution of Meiji γ-PGA (poly-γ-glutamic acid manufactured by Meiji Seika Kaisha, Ltd.) was placed in a glass tray. The solution was irradiated with a Cockcroft-Walton type electron beam irradiation device. The irradiation was performed from a distance of 10 cm, at 2.5kGy/1 sec, and for a total of 12 seconds so that the total irradiation dose would be 30 kGy. The treated material was immersed in water at 4 °C for 1 week to remove non-cross-linked poly-γ-glutamic acid. The swollen poly-γ-glutamic acid gel, by absorbing water, was filtered with an 80mesh wire-mesh filter. Then the gel was freeze-dried to obtain cross-linked poly-γ-glutamic acid with a gelation rate of 91 %.

### (Manufacturing example 2) Cross-linked poly aspartic acid

Lysine methyl ester dihydrochloride (7.2 parts) and lysine monohydrochloride (22.6 parts) were dissolved in distilled water (40 parts). The solution was neutralized by adding sodium hydroxide (7.8 parts) little by little to prepare a lysine aqueous solution. On the other hand, polysuccinimide (100 parts) with a weight-average molecular weight (Mw) of 96,000 was dissolved in DMF (400 parts) under nitrogen atmosphere. To this solution was added the aforementioned lysine aqueous solution, and the mixture was stirred for 1 hour. The reaction was continued for 20 hours after the stirring was stopped. The reactant was transferred to a mixer equipped with a stirrer with blades, and to the mixer was added distilled water (400 parts) and methanol (400 parts), and the gel was chopped at 8000 rpm for 5 minutes. Then, a 27 weight % sodium hydroxide aqueous solution (129.7 parts) was dropwise added during a span of 2 hours, the stirring was continued for 2 hours, and the mixture was neutralized with 7 weight % hydrochloric acid to pH 7. Subsequently, to the mixture was added methanol (300 parts), and the precipitate was dried at 60 °C. Cross-linked polyaspartic acid (143 parts) was obtained by crushing the dried precipitate with a sample mill until a particle diameter of less than 100 µm was reached.

Samples shown in the following Table 1, 2 are prepared, and they are evaluated by the above valuation basis.

**(Table 1)**

| | Test example | | | | | | | | | |
|---|---|---|---|---|---|---|---|---|---|---|
| | 1-1 | 1-2 | 1-3 | 1-4 | 1-5 | 1-6 | 2-1 | 2-2 | 2-3 | 2-4 |
| Components | | | | | | | | | | |
| (1) Thickener | | | | | | | | | | |
| | Acrylic polymer of this invention (*1) | | | | | | | | | |
| | (as polymer (30%)) | | | | | | | | | |
| | 0.1 | 0.5 | 1.0 | 5.0 | 10.0 | 30.0 | 1.5 | 1.5 | 10.0 | 10.0 |
| | (0.03) | (0.15) | (0.3) | (1.5) | (3.0) | (9.0) | (0.15) | (0.15) | (3.0) | (3.0) |
| | Carboxy vinyl polymer (*2) | | | | | | | | | |
| | - | - | 0.1 | - | - | - | 0.1 | - | 1.0 | - |
| | Alkyl denaturated carboxy vinyl polymer (*3) | | | | | | | | | |
| | - | - | - | - | 0.1 | - | - | - | - | - |
| | Xanthan glum | | | | | | | | | |
| | - | - | - | - | - | - | - | 0.1 | - | 1.0 |
| (2) | Cross-linked poly γ -sodium glutamate | | | | | | | | | |
| (Trade name: gel protein A-8001, 2% solution, manufactured by Idemitsu Technofine Co.) | | | | | | | | | | |
| | 25.0 | 50.0 | 30.0 | 10.0 | - | 50.0 | - | - | - | - |
| (3) | Cross-linked poly aspartic acid (Manufacturing example 2) | | | | | | | | | |
| | - | - | - | - | 2.5 | - | - | - | - | - |
| (4) | Bio yeast extract (*4) | | | | | | | | | |
| | - | 0.1 | 0.05 | 0.2 | 0.01 | 0.3 | 0.1 | 0.1 | 0.05 | 0.05 |
| (5) | 1,3-Butylene glycol | | | | | | | | | |
| | 8.0 | 8.0 | 8.0 | 8.0 | 8.0 | 8.0 | 8.0 | 8.0 | 8.0 | 8.0 |
| (6) | Paraben | | | | | | | | | |
| | 0.15 | 0.15 | 0.15 | 0.15 | 0.15 | 0.15 | 0.15 | 0.15 | 0.15 | 0.15 |
| (7) | EDTA-3Na | | | | | | | | | |
| | 0.01 | 0.01 | 0.01 | 0.01 | 0.01 | 0.01 | 0.01 | 0.01 | 0.01 | 0.01 |
| (8) | Potassium hydroxide | | | Proper quantity | | | | | | |
| (9) | Perfume | | | Proper quantity | | | | | | |
| (10) | Water | | | Remainder | | | | | | |
| (11) | POE (10 mol) monooleate | | | | | | | | | |
| | 1.0 | 1.0 | 1.0 | 1.0 | 1.0 | 1.0 | 1.0 | 1.0 | 1.0 | 1.0 |
| (12) | Glycerol monistearate | | | | | | | | | |
| | 1.0 | 1.0 | 1.0 | 1.0 | 1.0 | 1.0 | 1.0 | 1.0 | 1.0 | 1.0 |
| (13) | Cetyl alcohol | | | | | | | | | |
| | 1.0 | 1.0 | 1.0 | 1.0 | 1.0 | 1.0 | 1.0 | 1.0 | 1.0 | 1.0 |
| (14) | Bee wax | | | | | | | | | |
| | 0.5 | 0.5 | 0.5 | 0.5 | 0.5 | 0.5 | 0.5 | 0.5 | 0.5 | 0.5 |
| (15) | Vaseline | | | | | | | | | |
| | 2.0 | 2.0 | 2.0 | 2.0 | 2.0 | 2.0 | 2.0 | 2.0 | 2.0 | 2.0 |
| (16) | Squalane | | | | | | | | | |
| | 6.0 | 6.0 | 6.0 | 6.0 | 6.0 | 6.0 | 6.0 | 6.0 | 6.0 | 6.0 |
| (17) | Dimethylpolysiloxane (6mPa·s) | | | | | | | | | |
| | 2.0 | 2.0 | 2.0 | 2.0 | 2.0 | 2.0 | 2.0 | 2.0 | 2.0 | 2.0 |
| Activity of improving elasticity and alleviating wrinkles | | | | | | | | | | |
| | A | A | A | A | B | A | B | C | B | C |
| Moisturizing activity (moist feeling) | | | | | | | | | | |
| | A | A | A | A | B | A | C | C | C | C |
| Stickiness | | | | | | | | | | |
| | A | A | A | B | B | A | D | D | C | C |
| Spreadability on skin | | | | | | | | | | |
| | A | A | A | A | B | A | C | D | D | D |

**(Table 2)**

| | Test example | | | | | | | |
|---|---|---|---|---|---|---|---|---|
| | 3-1 | 3-2 | 3-3 | 3-4 | 3-5 | 3-6 | 3-7 | 3-8 |
| Components | | | | | | | | |
| (1) Thickener | | | | | | | | |
| | Carboxy vinyl polymer (*2) | | | | | | | |
| | 0.01 | 3.0 | 0.5 | - | - | - | - | - |
| | Alkyl denaturated carboxy vinyl polymer (*3) | | | | | | | |
| | - | - | 0.5 | 0.5 | - | - | - | - |
| | Xanthan gum | | | | | | | |
| | - | - | - | - | 0.01 | 3.0 | 0.5 | - |
| | Hydroxyethyl cellulose | | | | | | | |
| | - | - | - | - | - | - | 0.5 | 0.5 |
| (2) | Cross-linked poly γ -sodium glutamate | | | | | | | |
| (Trade name: gel protein A-8001, 2% solution, manufactured by Idemitsu Technofine Co.) | | | | | | | | |
| | 50.0 | - | - | - | 50.0 | - | - | - |
| (3) | Cross-linked poly aspartic acid (Manufacturing example 2) | | | | | | | |
| | - | - | - | 2.5 | - | - | - | 2.5 |
| (4) | Bio yeast extract (*4) | | | | | | | |
| | - | 0.2 | 0.3 | 0.01 | - | 0.2 | 0.3 | 0.01 |
| (5) | 1,3-Butylene glycol | | | | | | | |
| | 8.0 | 8.0 | 8.0 | 8.0 | 8.0 | 8.0 | 8.0 | 8.0 |
| (6) | Paraben | | | | | | | |
| | 0.15 | 0.15 | 0.15 | 0.15 | 0.15 | 0.15 | 0.15 | 0.15 |
| (7) | EDTA-3Na | | | | | | | |
| | 0.01 | 0.01 | 0.01 | 0.01 | 0.01 | 0.01 | 0.01 | 0.01 |
| (8) | Potassium hydroxide | | | | Proper quantity | | | |
| (9) | Perfume | | | | Proper quantity | | | |
| (10) | water | | | | remainder | | | |
| (11) | POE (10 mol) monooleate | | | | | | | |
| | 1.0 | 1.0 | 1.0 | 1.0 | 1.0 | 1.0 | 1.0 | 1.0 |
| (12) | Glycerol monostearate | | | | | | | |
| | 1.0 | 1.0 | 1.0 | 1.0 | 1.0 | 1.0 | 1.0 | 1.0 |
| (13) | Cetyl alcohol | | | | | | | |
| | 1.0 | 1.0 | 1.0 | 1.0 | 1.0 | 1.0 | 1.0 | 1.0 |
| (14) | Bee wax | | | | | | | |
| | 0.5 | 0.5 | 0.5 | 0.5 | 0.5 | 0.5 | 0.5 | 0.5 |
| (15) | Vaseline | | | | | | | |
| | 2.0 | 2.0 | 2.0 | 2.0 | 2.0 | 2.0 | 2.0 | 2.0 |
| (16) | Squalane | | | | | | | |
| | 6.0 | 6.0 | 6.0 | 6.0 | 6.0 | 6.0 | 6.0 | 6.0 |
| (17) | Dimethylpolysiloxane (6mPa·s) | | | | | | | |
| | 2.0 | 2.0 | 2.0 | 2.0 | 2.0 | 2.0 | 2.0 | 2.0 |
| Activity of improving elasticity and alleviating wrinkles | | | | | | | | |
| | D | D | C | C | D | D | C | C |
| Moisturizing activity (moist feeling) | | | | | | | | |
| | B | D | D | C | B | D | D | C |
| Stickiness | | | | | | | | |
| | C | C | D | B | C | D | D | D |
| Spreadability on skin | | | | | | | | |
| | C | C | D | C | D | D | C | C |

| | | | | | | | | |
|---|---|---|---|---|---|---|---|---|
| * 1 : ACULYN 22 (polymer 30%)(manufactured by Rohm and Haas Co.) * 2 : SYNTHALEN K(manufactured by 3V SIGMA Co.) * 3 : PEMULEN TR-1(manufactured by NOVEON Co.) * 4 : Biodynes EMPP (manufactured by Arch Personal Care Products L.P. Co.) | | | | | | | | |

### (Process)

A desired skin care cream was obtained by gradually adding the oil phase of (11)-(17) to the aqueous phase of (1)-(10) while the aqueous phase was being stirred with a dispersion mill.

In all examples in which acrylic polymers and cross-linked polyamino acids of the present invention were used, excellent effects in improving elasticity and alleviating wrinkles while moisturizing the skin were attained. Non-stickiness and good spreadability on the skin were also attained (Test examples 1-1 to 1-6). On the contrary, in the examples in which only component (A) is used (Test examples 2-1 to 2-4), or conventional thickeners are used instead of component (A) (Test examples 3-1 to 3-8), there were the following problems. The effect of improving elasticity and alleviating wrinkles was not satisfactory, and the moisturizing effect was not sufficient. The spreadability on the skin was also poor, and there was stickiness.

### <Activity of improving elasticity and alleviating wrinkles>

The activity of improving elasticity and alleviating wrinkles was tested by measuring the below-described dynamic viscoelasticity.

### (The test method of dynamic viscoelasticity)

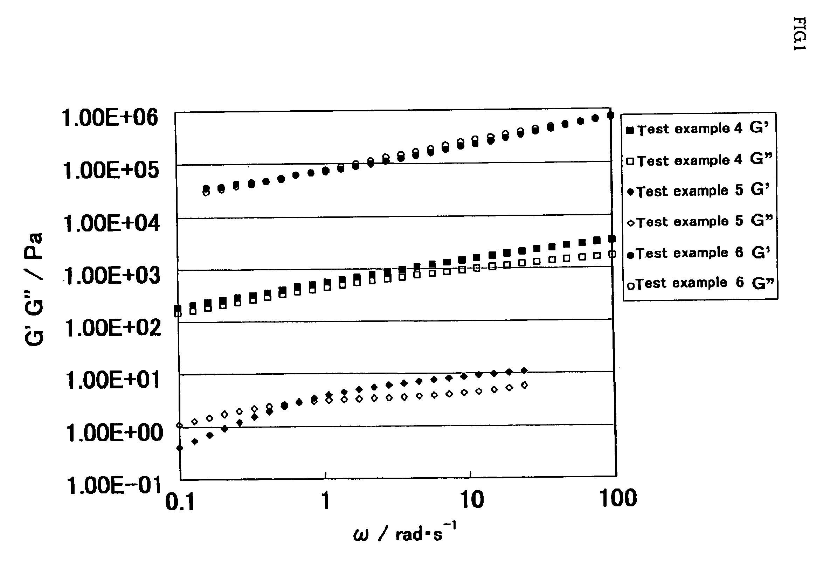

The storage modulus (G') and loss modulus (G") were measured with a corn-and-plate rheometer from Paar Physica (MCR-30) in the frequency range of 0.1 to 100 (rad/sec) for the samples of the below-described Test examples 4 to 6. The results are shown in Figure 1.
Test example 4: 3.3% aqueous solution of acrylate/steareth-20 methacrylate copolymer (trade name; ACULYN 22, manufactured by Rohm and Haas Co.) (polymer 1%). (pH is adjusted to 7 by potassium hydroxide).
Test example 5: 10% aqueous solution of cross-linked sodium poly-gamma-glutamic acid (trade name; gel protein A-8001, manufactured by Idemitsu Technofine Co.) (polymer 0.2%)
Test example 6: mixture of Test example 4 and Test example 5 (1:1).

As shown in Figure 1, the elastic modulus for the case in which component (A) and component (B) combined (Test example 6) were synergistically larger than those for independent individual cases (Test examples 4 and 5). Therefore, the excellent activity of improving elasticity of the composition in this invention was confirmed by measuring dynamic viscoelasticity.

As an additional experiment, 1 g sample of Test example 6 was dropped on a 3 cm square vitro skin (artificial skin) that had 76µm deep sulcus cutis. The vitro skin was allowed to stand for 24 hours at 25 °C under 50% RH. Then, the observation was conducted with a confocal microscope (HD100D) from Laser Tech.

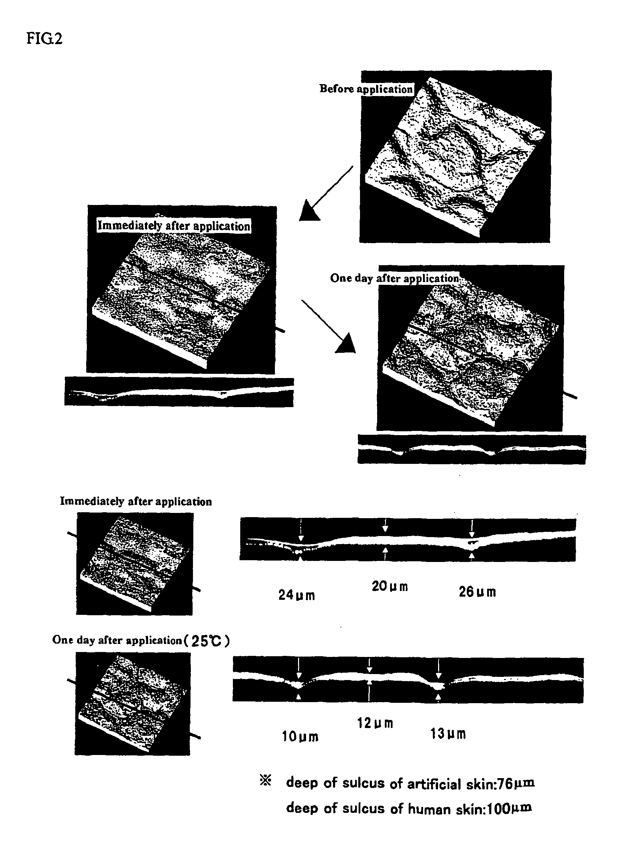

As shown in Figure 2, the case in which component (A) and component (B) were combined had decreased skin unevenness even one day after application. Thus, it was confirmed that the external composition for skin of the present invention had an excellent effect of alleviating wrinkles.

### <Moisturizing activity>

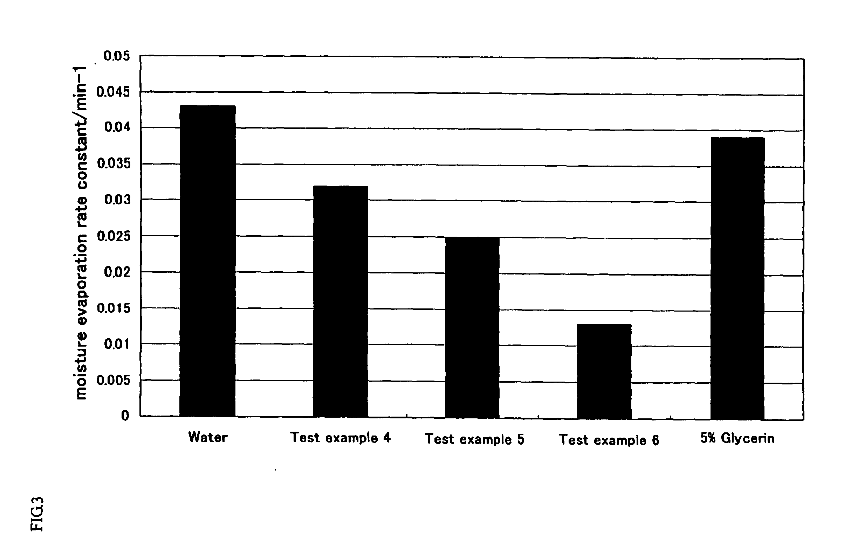

10 µl samples of Test examples 4-6, 10 µl of water, and 10 µl of 5% glycerin aqueous solution were dropped on respective filter papers of 2 cm square. The weight change of each filter paper was measured every 5 minutes at 25°C under 50% RH. The value n in (nX + m), which was derived by the least squares method applied for the results, was defined as the moisture evaporation rate constant. The average of three values for each sample was plotted as the absolute value. The lower the moisture evaporation rate constant, the more moisturizing effect it has. The results are shown in Figure 3.

As shown in Figure 3, the moisturizing effect for the case in which component (A) and component (B) were combined (Test example 6) was drastically better than that for independent individual cases (Test examples 4 and 5). Even when compared with the conventional moisturizing agent glycerin, Test example 6 had a significantly higher moisturizing effect. Therefore, the excellent moisturizing activity of the composition in this invention was confirmed by measuring moisture evaporation rate constant.

| <Skin cream> | |
|---|---|
| (Blending components) | (weight %) |
| (1) 1,3-Butylene glycol | 5.0 |
| (2) (Sodium acrylate / acryloyl dimethyl taurine) copolymer 1.0 | |
| (Trade name : SIMULGEL EG, manufactured by SEPIC Co.) | |
| (3) Cross-linked poly-gammaglutamic acid | 3.0 |
| (Manufacturing example 1) | |
| (4) Polyethylene glycol (40E.O.) monostearate | 1.5 |
| (Trade name ; NIKKOL MYS-40V, manufactured by Nikko chemicals Co.) | |
| (5) Glyceryl monostearate (self emulsion type) | 2.5 |
| (Trade name : NIKKOL MGS-ASE, manufactured by Nikko chemicals Co.) | |
| (6) Hydrogenated poly isobutene | 5.0 |
| (7) Meadowfoam oil | 5.0 |
| (8) Decamethyl cyclopentasiloxane | 3.0 |
| (9) Stearyl alcohol | 0.8 |
| (10) Behenyl alcohol | 1.5 |
| (11) Cetanol | 4.0 |
| (12) Microcrystalline wax | 2.0 |
| (13) Acrylate / beheneth-25 methacrylate copolymer | 3.0 |
| (Trade name : ACULYN 28, manufactured by Rohm and Haas Co.) | |
| (14) Potassium hydroxide | 0.1 |
| (15) Paraben | proper quantity |
| (16) Edetic salt | proper quantity |
| (17) Dibutyl hydroxytoluene | proper quantity |
| (18) Perfume | proper quantity |
| (19) Purified water | remainder |

### (Process)

Components (1)-(3), (13)-(17), and (19) were evenly mixed and dissolved, and this water phase was warmed to 70 °C. Components (4)-(12) and (18) were dissolved by heating to 70 °C to form uniform oil phase. The oil phase was gradually added to the water phase, and the mixture was emulsified by a homo mixer. Subsequently, the emulsion was cooled with a heat exchanger, and the desired cream was obtained. The pH of obtained skin care cream was 7.0.

This article has excellent effects in improving elasticity and alleviating wrinkles while moisturizing the skin, has good spreadability on the skin, and has no stickiness.

### <Skin care milky lotion>

| (Blending components) | (weight %) |
|---|---|
| (1) 1,3-Butylene glycol | 4.0 |
| (2) Dynamite glycerin | 7.0 |
| (3) Polyethylene glycol 1500 | 1.5 |
| (4) Polyethylene glycol 20,000 | 1.0 |
| (5) Xylitol | 1.0 |
| (6) Acetylated hyaluronic acid | 0.01 |
| (7) Carboxyvinyl polymer | 0.14 |
| (8) Xanthan gum | 0.05 |
| (9) Sodium hydroxide | 0.2 |
| (10) Acrylate/steareth-20 methacrylate copolymer | 1.0 |
| (Trade name:ACULYN 22 (polymer 30%), manufactured by Rohm and Haas Co.) | |
| (11) Cross-linked poly-gamma-glutamic acid | 1.0 |
| (Manufacturing example 1) | |
| (12) Polyethylene glycol-60 glyceryl isostearate | 1.2 |
| (Trade name : EMALEX GWIS-160, manufactured by Japanese emulsion Co.) | |
| (13) Polyethylene glycol-5 glyceryl stearate | 0.5 |
| (Trade name : EMALEX GM-5, manufactured by Japanese emulsion Co.) | |
| (14) Stearic acid | 0.45 |
| (15) Myristic acid | 0.15 |
| (16) Hydrogenated palm oil | 2.0 |
| (17) Squalane | 1.0 |
| (18) Pentaerythritol tetra-2-ethyl hexanoate | 4.5 |
| (19) Dimethylpolysiloxane (6mPa s) | 2.0 |
| (20) Rosa Roxburghii Extract | 0.02 |
| (21) L-serine | 0.01 |
| (22) Trimethyl glycine | 1.0 |
| (23) Zingiber Aromaticus Extract | 0.3 |
| (24) Tranexamic acid | 2.0 |
| (25) Vitamins-E acetate | 0.05 |
| (26) Sodium hexametaphosphate (first class grade chemical) | 0.01 |
| (27) Ethyl paraben | 0.23 |
| (28) Butyl paraben | 0.1 |
| (29) Iron oxide | proper quantity |
| (30) Paraben | proper quantity |
| (31) Perfume | proper quantity |
| (32) Purified water | remainder |

### (Process)

Components (1)-(11), (20)-(30), and (32) were evenly mixed and dissolved, and this water phase was warmed to 70 °C. Components (4)-(12) and (18) were dissolved by heating to 70 °C to form uniform oil phase. The oil phase was gradually added to the water phase, and the mixture was emulsified by a homo mixer. Subsequently, the emulsion was cooled with a heat exchanger, and the desired skin care milky lotion was obtained. The pH of obtained skin care milky lotion was 6.5.

This article has excellent effects in improving elasticity and alleviating wrinkles while moisturizing the skin, has good spreadability on the skin, and has no stickiness.

### <Skin care cream>

| (Blending components) | (weight %) |
|---|---|
| (1) Stearic acid | 8.0 |
| (2) Stearyl acohol | 4.0 |
| (3) Butyl stearate | 6.0 |
| (4) Propylene glycol | 5.0 |
| (5) Cross-linked poly-gamma-glutamic acid | 10.0 |
| (Trade name;gel protein A-8001) | |
| (6) Glycerol monostearate | 2.0 |
| (7) Potassium hydroxide | 0.4 |
| (8) Acrylate/beheneth-20 methacrylate copolymer | 1.5 |
| (Trade name; ACULYN 22 (polymer 30%), manufactured by Rohm and Haas Co.) | |
| (9) Methyl paraben | 0.15 |
| (10) Butyl hydroxy toluene | proper quantity |
| (11) Perfume | proper quantity |
| (12) Purified water | remainder |
| (13) L-ascorbic acid | 1.0 |

### (Process)

Components (4), (5), (7), (8), (9), and (13) were added to component (12) and warmed to 70°C. Components (1)-(3), (6), (10), and (11) were dissolved by heating to 70°C and added to the above water phase. Then preliminary emulsification was carried out. After emulsified particles were homogenized with a homo mixer, the skin care cream was obtained by aeration, filtration, and cooling. The pH of obtained skin care cream was 6.3.

This article has excellent effects in improving elasticity and alleviating wrinkles while moisturizing the skin, has good spreadability on the skin, and has no stickiness.

### <Skin care milky lotion>

| (Blending components) | (weight %) |
|---|---|
| (1) Stearic acid | 2.0 |
| (2) Cetyl alcohol | 1.0 |
| (3) Vaseline | 4.0 |
| (4) Squalane | 5.0 |
| (5) Glycerol tri-2-ethyl hexanoate | 2.0 |
| (6) Sorbitan monooleate | 2.0 |
| (7) Dipropylene glycol | 5.0 |
| (8) Polyethylene glycol 1500 | 3.0 |
| (9) Triethanolamine | 1.0 |
| (10) Cross-linked sodium poly-gamma-glutamate | 5.0 |
| (Trade name: gel protein A-8001, manufactured by Idemitsu Technofine Co.) | |
| (11) Ethyl paraben | 0.2 |
| (12) Perfume | proper quantity |
| (13) Purified water | remainder |
| (14) Acrylate/beheneth-25 methacrylate copolymer | 1.0 |
| (Trade name; ACULYN 28 (polymer 20%), manufactured by Rohm and Haas Co.) | |
| (15) Sodium hydroxide | 0.1 |
| (16) Rose fruit extract | 0.2 |
| (17) Scutellaria baicalensis extract | 0.1 |

### (Process)

Components (7), (8), (9), (10), (14) and (15)-(17) were added to component (14) and warmed to 70°C (water phase). Components (1)-(6), (11) and (12) were mixed and heated to 70°C (oil phase). The oil phase was added to the above water phase and preliminary emulsification was carried out. After emulsified particles were homogenized with a homo mixer, the skin care milky lotion was obtained by aeration, filtration, and cooling. The pH of obtained skin care milky lotion was 6.3.

This article has excellent effects in improving elasticity and alleviating wrinkles while moisturizing the skin, has good spreadability on the skin, and has no stickiness.

### <Skin care milky lotion>

| (Blending components) | (weight %) |
|---|---|
| (1) Cetyl alcohol | 1.0 |
| (2) Bee wax | 0.5 |
| (3) Vaseline | 2.0 |
| (4) Squalane | 6.0 |
| (5) Dimethylpolysiloxane | 2.0 |
| (6) Ethanol | 5.0 |
| (7) Glycerol | 4.0 |
| (8) 1,3-Butylene glycol | 4.0 |
| (9) Cross-linked sodium poly-gamma-glutamate | 0.5 |
| (Trade name; gel protein A-8001, manufactured by Idemitsu Technofine Co.) | |
| (10) Polyoxyethylene (10) monooleate | 1.0 |
| (11) Glycerol monostearate | 1.0 |
| (12) Quince seed extract (5% of aqueous solution) | 5.0 |
| (13) Phenoxyethanol | 0.2 |
| (14) Color agent (water-soluble dye) | proper quantity |
| (15) Perfume | proper quantity |
| (16) Purified water | remainder |
| (17) Acrylate/steareth-20 methacrylate copolymer | 0.2 |
| (Trade name; ACULYN 22 (polymer 30%), manufactured by Rohm and Haas Co.) | |
| (18) Triethanolamine | 0.1 |
| (19) Gambir extract | 0.3 |

### (Process)

Components (6)-(9), (12)-(14), (17), (18) and (19) were added to component (16) and warmed to 70°C (water phase). Components (1)-(5), (10), (11) and (15) wer e mixed and heated to 70°C (oil phase). The oil phase was added to the above water phase and preliminary emulsification was carried out. After emulsified particles were homogenized with a homo mixer, the skin care milky lotion was obtained by aeration, filtration, and cooling. The pH of obtained skin care milky lotion was 6.8.

This article has excellent effects in improving elasticity and alleviating wrinkles while moisturizing the skin, has good spreadability on the skin, and has no stickiness.

### <Lotion>

| (Blending components) | (weight %) |
|---|---|
| (1) 1,3-Butylene glycol | 6.0 |
| (2) Glycerol | 4.0 |
| (3) Oleyl alcohol | 0.1 |
| (4) Polyoxyethylene (20) sorbitan monolaurate ester | 0.5 |
| (5) Polyoxyethylene (15) lauryl alcohol ether | 0.5 |
| (6) Ethanol | 10.0 |
| (7) Cross-linked sodium poly-gamma-glutamate | 1.0 |
| (Trade name; gel protein A-8001, manufactured by Idemitsu Technofine Co.) | |
| (8) Perfume | proper quantity |
| (9) Color agent (water-soluble dye) | proper quantity |
| (10) Methyl paraben | 0.2 |
| (11) Fading inhibitor (dimorpholino pyridazinone) proper quantity | |
| (12) Sodium citrate | 0.04 |
| (13) Citric acid | 0.06 |
| (14) Acrylate/beheneth-25 methacrylate copolymer | 0.1 |
| (Trade name; ACULYN 28, manufactured by Rohm and Haas Co.) | |
| (15) Sodium hydroxide | 0.3 |
| (16) Purified water | remainder |
| (17) 2-O-alpha-D-glucopyranosyl-L-ascorbic acid | 2.0 |

### (Process)

Components (1), (2), (7), (9), (11)-(15), and (17) were dissolved in (16) at room temperature to obtain a water phase. Components (3)-(5), (8), and (10) were dissolved in (6), and this solution was mixed with the above water phase and solubilized. Thus, the desired lotion was obtained. The pH of obtained lotion was 6.7.

This article has excellent effects in improving elasticity and alleviating wrinkles while moisturizing the skin, has good spreadability on the skin, and has no stickiness.

### <Microemulsion type lotion>

| (Blending components) | (weight %) |
|---|---|
| (1) 1,3-Butylene glycol | 6.0 |
| (2) Glycerin | 5.0 |
| (3) Polyethylene glycol 4000 | 3.0 |
| (4) Cross-linked sodium poly-gamma-glutamate | 3.0 |
| (Trade name; gel protein A-8001, manufactured by Idemitsu Technofine Co.) | |
| (5) Acrylate/steareth-20 methacrylate copolymer | 0.5 |
| (Trade name; ACULYN 22, manufactured by Rohm and Haas Co.) | |
| (6) Potassium hydroxide | 0.1 |
| (7) Olive oil | 0.5 |
| (8) Polyoxyethylene (20) sorbitan monostearate | 1.5 |
| (9) Polyoxyethylene (5) oleil alcohol ether | 0.3 |
| (10) Ethanol | 10.0 |
| (11) Perfume | proper quantity |
| (12) Color agent (water-soluble dye) | proper quantity |
| (13) Methyl paraben | 0.3 |
| (14) Lactic acid | 0.06 |
| (15) Sodium lactate | 0.04 |
| (16) Edetic salt | proper quantity |
| (17) Purified water | remainder |
| (18) Rose extract | 0.1 |

### (process)

Components (4), (5), (6) were dissolved in a portion of component (17) (part of water phase). Subsequently, components (1)-(3), (12), (14)-(16), and (18) were added to the rest of component (17) and dissolved at room temperature (water phase). On the other hand, components (7)-(9), (11), and (13) were added to component (10) and dissolved at room temperature. This alcohol phase was added to the above water phase, and a microemulsion was prepared. To this microemulsion was added the first part of water phase, and thus the desired microemulsion type lotion was obtained. The pH of obtained microemulsion type lotion was 7.0.

This article has excellent effects in improving elasticity and alleviating wrinkles while moisturizing the skin, has good spreadability on the skin, and has no stickiness.

### <Skin care cream>

| (Blending components) | (weight %) |
|---|---|
| (1) Cetyl alcohol | 5.0 |
| (2) Stearic acid | 3.0 |
| (3) Vaseline | 5.0 |
| (4) Squalane | 10.0 |
| (5) Glycerol tri 2-ethyl hexanoate | 7.0 |
| (6) Dipropylene glycol | 5.0 |
| (7) Glycerin | 5.0 |
| (8) Cross-linked sodium poly-gamma-glutamate | 15.0 |

| (Trade name; gel protein A-8001, manufactured by Idemitsu Technofine Co.) | |
|---|---|
| (9) Propylene glycol monostearate | 7.0 |
| (10) Polyoxyethylene (20) cetyl alcohol ether | 3.0 |
| (11) Triethanolamine | 1.0 |
| (12) Butyl paraben | 0.15 |
| (13) Butyl hydroxy toluene | proper quantity |
| (14) Perfume | proper quantity |
| (15) Purified water | remainder |
| (16) Acrylate/steareth-20 methacrylate copolymer 2.0 | |

| (Trade name; ACULYN 22 (polymer 30%), manufactured by Rohm and Haas Co.) | |
|---|---|
| (17) Saxifraga extract | 0.1 |
| (18) Thymus extract | 0.1 |
| (19) Sodium hydroxide | 0.1 |

### (Process)

Components (6)-(8), (11), and (16)- (19) were added to component (15) and warmed to 70°C (water phase). Components (1)-(5), (10), (12), (13) and (14) were mixed and heated to 70°C (oil phase). The oil phase was added to the above water phase and preliminary emulsification was carried out. After emulsified particles were homogenized with a homo mixer, the skin care cream was obtained by aeration, filtration, and cooling. The pH of obtained skin care cream was 6.2.

This article has excellent effects in improving elasticity and alleviating wrinkles while moisturizing the skin, has good spreadability on the skin, and has no stickiness.

The external composition for skin of this invention has excellent effects in improving elasticity and alleviating wrinkles while moisturizing the skin, has good spreadability on the skin, and has no stickiness by using a specific acrylic polymer as a thickener and by blending a cross-linked polyamino acid.

## Claims

1. An external composition for skin for improving elasticity and alleviating wrinkles comprising
(A) one or more selected from acrylate/ceteth-20 methacrylate copolymer, acrylate/steareth-50 methacrylate copolymer, acrylate/steareth-20 methacrylate copolymer, and acrylate/beheneth-25 methacrylate copolymer and,
(B) one or more selected from cross-linked poly-gamma-glutamic acid, cross-linked poly-aspartic acid and their salts.

2. The external composition for skin for improving elasticity and alleviating wrinkles according to claim 1,
wherein comprising 0.01 to 10.0 weight-% of said component (A) as a polymer, and 0.0001 to 5.0 weight-% of component (B) as a polymer.

3. The external composition for skin for improving elasticity and alleviating wrinkles according to any of claims 1 to 2, wherein comprising a water-soluble drug.

4. The external composition for skin for improving elasticity and alleviating wrinkles according to any of claims 1 to 3 wherein pH is 6.0 to 7.5.

5. The use of the external composition for skin according to any of claims 1 to 4 for improving elasticity and alleviating wrinkles.

## Patentansprüche

1. Äußerlich anzuwendende Zusammensetzung für die Haut zur Verbesserung der Elastizität und zur Milderung von Falten, umfassend:
(A) eine oder mehrere Komponenten, ausgewählt aus einem Acrylat/Ceteth-20-Methacrylat-Copolymer, einem Acrylat/Steareth-50-Methacrylat-Copolymer, einem Acrylat/Steareth-20-Methacrylat-Copolymer und einem Acrylat/Beheneth-25-Methacrylat-Copolymer und
(B) eine oder mehrere Komponenten, ausgewählt aus vernetzter Poly-Gamma-Glutaminsäure, vernetzter Polyasparaginsäure und ihren Salzen.

2. Äußerlich anzuwendende Zusammensetzung für die Haut zur Verbesserung der Elastizität und zur Milderung von Falten nach Anspruch 1,
umfassend 0,01 bis 10,0 Gew.-% der Komponente (A) als Polymer und 0,0001 bis 5,0 Gew.-% der Komponente (B) als Polymer.

3. Äußerlich anzuwendende Zusammensetzung für die Haut zur Verbesserung der Elastizität und zur Milderung von Falten nach einem der Ansprüche 1 bis 2, umfassend ein wasserlösliches Arzneimittel.

4. Äußerlich anzuwendende Zusammensetzung für die Haut zur Verbesserung der Elastizität und zur Milderung von Falten nach einem der Ansprüche 1 bis 3, wobei der pH-Wert 6,0 bis 7,5 beträgt.

5. Verwendung der äußerlich anzuwendenden Zusammensetzung für die Haut nach einem der Ansprüche 1 bis 4 zur Verbesserung der Elastizität und zur Milderung von Falten.

## Revendications

1. Une composition externe pour la peau pour améliorer l'élasticité et soulager les rides comprenant
(A) un ou plusieurs copolymères sélectionnés à partir de copolymère méthacrylate acrylate/ceteth-20, copolymère méthacrylate acrylate/steareth-50, copolymère méthacrylate acrylate/steareth-20, et copolymère méthacrylate acrylate/beheneth-25 et,
(B) un ou plusieurs acides sélectionnés à partir d'acide poly-gamma-glutamique réticulé, acide poly-aspartique réticulé et leurs sels.

2. La composition externe pour la peau pour améliorer l'élasticité et soulager les rides selon la revendication 1,
Où cela comprend 0,01 à 10,0 de pourcentage en poids dudit composant (A) en tant que polymère, et 0,0001 à 5,0 de pourcentage en poids du composant (B) en tant que polymère.

3. La composition externe pour la peau pour améliorer l'élasticité et soulager les rides selon l'une des revendications 1 à 2, où cela comprend une substance soluble dans l'eau.

4. La composition externe pour la peau pour améliorer l'élasticité et soulager les rides selon l'une des revendications 1 à 3, où le pH_est 6,0 à 7,5.

5. L'utilisation de la composition externe pour la peau selon l'une des revendications 1 à 4 pour améliorer l'élasticité et soulager les rides.
